(19) Europäisches Patentamt
European Patent Office
Office européen des brevets

(11) Veröffentlichungsnummer : **0 346 733 B1**

(12) # EUROPÄISCHE PATENTSCHRIFT

(45) Veröffentlichungstag der Patentschrift : **15.04.92 Patentblatt 92/16**

(51) Int. Cl.[5] : **C07C 215/48,** C07C 215/76, C07C 213/00, A01N 33/10

(21) Anmeldenummer : **89110210.5**

(22) Anmeldetag : **06.06.89**

(54) **Aminomethylphenole und diese enthaltende Fungizide.**

(30) Priorität : **15.06.88 DE 3820292**

(43) Veröffentlichungstag der Anmeldung : **20.12.89 Patentblatt 89/51**

(45) Bekanntmachung des Hinweises auf die Patenterteilung : **15.04.92 Patentblatt 92/16**

(84) Benannte Vertragsstaaten : **AT BE CH DE ES FR GB GR IT LI NL SE**

(56) Entgegenhaltungen :
**DD-A- 108 974**
**DE-C- 1 164 152**
**US-A- 2 795 613**

(73) Patentinhaber : **BASF Aktiengesellschaft**
**Carl-Bosch-Strasse 38**
**W-6700 Ludwigshafen (DE)**

(72) Erfinder : **Rentzea, Costin, Dr.**
**Richard-Kuhn-Strasse 1-3**
**W-6900 Heidelberg (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

**EP 0 346 733 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue, substituierte o-Aminomethylphenole mit fungizider Wirkung, deren Herstellung, Fungizide, welche diese Phenole als Wirkstoffe enthalten, sowie deren Verwendung zur Bekämpfung von Schadpilzen.

Es ist bekannt, N-Tridecyl-2,6-dimethylmorpholin als Fungizid zu verwenden (DE-11 64 152). Es ist ferner die Verbindung N-(4-tert.-Butyl-cyclohexyl)-3,5-dibrom-2-hydroxy-benzylamin ohne Hinweis auf eine pflanzenfungizide Wirkung bekannt (DD-A-108 974).

Es wurde nun gefunden, daß substituierte o-Aminomethylphenole der Formel I

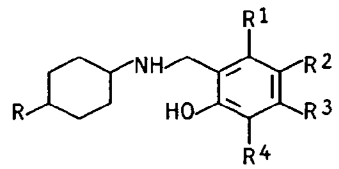

I

worin

$R^1$, $R^3$, $R^4$ Wasserstoff, Fluor, Chlor, Brom, $(C_1$-$C_4)$-Alkyl oder $(C_1$-$C_4)$-Alkoxy oder Dimethylamino bedeutet und,

$R^2$ Wasserstoff, Chlor, Brom, $(C_1$-$C_{10})$-Alkyl, $(C_3$-$C_8)$-Alkenyl, Alkoxyalkyl, Cyclohexyl oder Phenyl bedeutet oder

$R^1$ mit $R^2$, $R^2$ mit $R^3$ oder $R^3$ mit $R^4$ einen carbocyclischen, gesättigten oder ungesättigten Fünf- oder Sechsring bildet und

R $(C_1$-$C_5)$-Alkyl bedeutet, das gegenüber dem Stickstoffatom sowohl trans- als auch cis-konfiguriert sein kann, und deren Salze ausgezeichnet wirksam gegen Schadpilze sind und gute Pflanzenverträglichkeit zeigen.

Die Reste $R^1$, $R^2$, $R^3$ und $R^4$ können gleich oder verschieden sein.

Die neuen o-Aminomethylphenole der Formel I enthalten ggf. chirale Zentren. Sie werden im allgemeinen als Racemate und gegebenenfalls als Diastereomerengemische erhalten. Einheitlich konfigurierte Isomere lassen sich durch bekannte Methoden isolieren. Alle diese Verbindungen und Gemische werden von der vorliegenden Erfindung umfaßt. Für die Anwendung der neuen o-Aminomethylphenole als Fungizide sind sowohl die einheitlichen Diastereomeren, Enantiomeren bzw. cis/trans-Isomeren wie auch deren bei der Synthese anfallenden Gemische geeignet. Bevorzugt werden die letzteren verwendet.

$R^1$ ist $C_1$-$C_4$-Alkyl bedeutet beispielsweise Methyl, Ethyl, Propyl, Butyl.

$R^1$ ist $C_1$-$C_4$-Alkoxy bedeutet beispielsweise Methoxy, Ethoxy, Propoxy, Butoxy.

$R^2$ ist $C_1$-$C_{10}$-Alkyl bedeutet beispielsweise Methyl, Ethyl, Propyl, Butyl, Pentyl, Hexyl, Heptyl, Octyl, Nonyl, Decyl, insbesondere tert.-Butyl.

$R^2$ ist $C_3$-$C_8$-Alkenyl bedeutet beispielsweise Allyl, Methallyl, Butenyl, Alkoxyalkyl bedeutet beispielsweise $C_1$-$C_4$-Alkoxy-$C_1$-$C_4$-Alkyl, z.B. Methoxymethyl, Methoxyethyl, Ethoxyethyl.

$R^1$ bildet mit $R^2$ einen carbocyclischen, gesättigten oder ungesättigten Fünf- oder Sechsring ist so zu verstehen, daß der Ring zusammen mit den beiden Kohlenstoffatomen gebildet wird, deren Substitutenten $R^1$ und $R^2$ sind. Wenn $R^1$ und $R^2$ beispielsweise den Rest $-C_3H_6-$ bedeuten, dann bilden sie zusammen mit den beiden obengenannten Kohlenstoffatomen einen Fünfring mit 5 Kohlenstoffatomen im Ring.

R ist $C_1$-$C_5$-Alkyl bedeutet beispielsweise Methyl, Ethyl, Propyl, Butyl, Pentyl, insbesondere tert.-Butyl.

Die o-Aminomethylphenole der Formel I lassen sich z.B. herstellen, indem man

a) einen Salicylaldehyd der Formel II mit einem Amin der Formel III

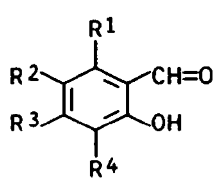

+

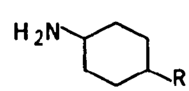

II III

in welcher R, $R^1$, $R^2$, $R^3$ und $R^4$ die oben genannten Bedeutungen haben, in Gegenwart von Ameisensäure, von Natriumcyanborhydrid, von Natriumborhydrid oder in Gegenwart von Wasserstoff und einem Hydrierkatalysator wie Ni, Pd oder Pt umsetzt, oder

b) ein Phenol der Formel IV mit Formaldehyd und mit einem Amin der Formel III

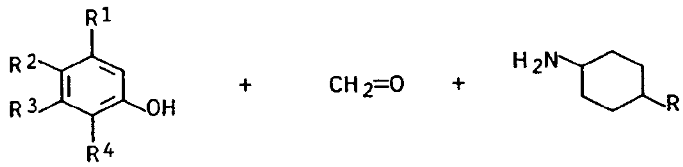

IV

III

in welcher R, $R^1$, $R^2$, $R^3$ und $R^4$ die oben genannten Bedeutungen haben, in Gegenwart eines basischen Katalysators umsetzt und die erhaltenen Verbindungen gegebenenfalls in ihre Salze überführt.

Gemäß Verfahrensvariante a) werden Amine der Formel III mit Aldehyden der Formel II z. B. in Gegenwart von Ameisensäure umgesetzt. Diese reduktive Alkylierung wird vorzugsweise in Abwesenheit eines Lösungsmittels durchgeführt. Zu einer Lösung des Amins in Ameisensäure wird bei Temperaturen von 0°C bis 110°C, vorzugsweise 50 bis 100°C der Aldehyd zugetropft.

Die Umsetzungen der Aldehyde mit Aminen der Formel III in Gegenwart von Natriumborhydrid oder Natriumcyanborhydrid werden z.B. in einem Lösungs- oder Verdünnungsmittel durchgeführt. Dazu eigenen sich vorzugsweise Alkohole wie Methanol, Ethanol, Propanol und Isopropanol, die bis zu 25 % (Vol.%) Wasser enthalten.

Gemäß Verfahrensvariante a) können Amine der Formel III mit Aldehyden der Formel II auch in Gegenwart von Wasserstoff und einem Hydrierkatalysator alkyliert werden, beispielsweise bei einem Wasserstoffdruck von 1 bis 150 bar und einer Temperatur von 25 bis 120°C.

Als Katalysatoren eigenen sich Edelmetalle wie beispielsweise Palladium, Platin - gegebenenfalls auf einem Träger niedergeschlagen - sowie Rhodium und Nickel (Raney-Nickel). Bevorzugt wird Palladium auf Kohle. Geeignete Lösungsmittel sind Alkohole wie Methanol oder Ethanol, Kohlenwasserstoffe wie Hexan, Heptan, Octan, Cyclohexan, Toluol oder Xylol.

Für diese Verfahrensvariante kommen als Lösungs- oder Verdünnungsmittel beispielsweise Halogenkohlenwasserstoffe, insbesondere Chlorkohlenwasserstoffe, z. B. Tetrachlorethylen-1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z. B. Ethylpropylether, Methyl-tert.-Butylether, n-Butylethylether, Di-n-butylether, Diisobutylether, Di-isobutylether, Diisoamylether, Diisopropylether, Anisol, Phenethol, Cyclohexylmethylether, Ethylenglykoldimethylether, Tetrahydrofuran, Dioxan, Thioanisol, β,β,β'-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyrnitril, Benzonitril, m-Chlorbenzonitril, aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2, 2, 4-Trimethylpentan, 2, 2, 3-Trimethylpentan, 2, 3, 3-Trimethylpentan, Octan; Ester z. B. Ethylacetat, Acetessigester, Isobutylacetat; Amide, z. B. Formamid, Methylformamid, Dimethylformamid; Ketone, z. B. Aceton, Methylethylketon, gegebenenfalls auch Wasser und entsprechende Gemische in Betracht.

Für die Verfahrensvariante b) kommen folgende Basen in Frage:

Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magesiumacetat, Zinkhydroxid, Zinkoxid, Zinkcarbonat, Zinkbicarbonat, Zinkacetat, Natriumformiat, Natriumacetat, Trimethylamin, Tripropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidin, N-Ethylpyrrolidion, N-Methylimidazol, N-Ethylimidazol, N-Methylhexamethylenimin, N-Ethylhexamethylenimin, Pyridin, Chinolin, α-Picolin, β-Picolin, α-Picolin, Isochinolin, Pyrimidin, Acridin, N,N,N',N'-Tetramethylethylendiamin, N,N',N',N-Tetraethylethylendiamin, Chinoxalin, Chinazolin, N-Propyldiisopropylamin, N,N-Dimethylcyclohexylamin, 2,6-Lutidin, 2,4-Lutidin, Trifurylamin, Triethylendiamin.

Hierzu werden vorzugsweise Lösungsmittel wie Wasser, Methanol, Ethanol, Tetrahydrofuran, Dioxan oder

deren Gemische verwendet.

Die Verfahrensvariante b) wird beispielsweise bei 10 bis 150°C durchgeführt.

Zur Salzbildung mit Verbindungen der Formel I sind alle organischen und anorganischen Säuren geeignet, soweit sie pflanzenphysiologisch verträgliche Salze bilden. So sind z. B. Chloride, Bromide, Jodide, Sulfate, Phosphate, Acetate, Oxalate, Fumarate, Malonate, Alkylsulfonate, Arylsulfonate und Dodecylbenzylsulfonate zu nennen.

Die Salze werden erhalten, indem man die entsprechende Säure mit freiem Amin der Formel I, gegebenenfalls in einem inerten Lösungsmittel zusammengibt, das Lösungsmittel abtrennt und den Rückstand gegebenenfalls umkristallisiert.

Die Ausgangsstoffe der Formel II, III und IV sind bekannt und/oder lassen sich nach an sich bekannten Methoden darstellen.

Die folgenden Beispiele erläutern die Herstellung der Verbindung der Formel I:

Beispiel 1

trans-2-(4′ -tert.-Butylcyclohexylamino)-methyl-4-tert.-pentyl-phenol

300 ml Dioxan werden zwischen 20 und 35 °C nacheinander mit 41,3 ml (0, 55 Mol) 37 %iger, wäßriger Formaldehydlösung, 85, 3g (0, 55 Mol) trans4-tert.-Butylcyclohexylamin, mit 95 g (0,579 Mol) 4-tert.-Pentylphenol und mit 1g Natriumhydroxid versetzt. Das Gemisch wird anschließend 6 Stunden bei 70°C gerührt, auf +5°C abgekühlt, der Niederschlag abgesaugt und mit 100 ml Wasser, dann mit 50 ml Dioxan gewaschen und getrocknet. Man erhält 104 g (57 d. Th.) trans-2-(4)-tert.-Butylcyclohexylamino)methyl-4-tert.pentyl-phenol als weiße Kristalle vom Schmp. 148-150°C (Wirkstoff Nr. 1).

Entsprechend können durch Wahl der Ausgangsstoffe und entsprechende Anpassung der Verfahrensbedingungen die nachstehend aufgelisteten Verbindungen der Formel I erhalten werden:

| Beisp. Nr. | R (trans/cis) $-C(CH_3)_3$ | $R^1$ | $R^2$ | $R^3$ | $R^4$ | Salz (Säure) | Fp °C |
|---|---|---|---|---|---|---|---|
| 2 | trans | H | $-CH_2-CH_2-CH_2-$ | | H | – | 128-130 |
| 3 | trans | H | $-C(CH_3)_3$ | H | H | – | 161-163 |
| 4 | trans | H | $-C(CH_3)_3$ | H | $-CH_3$ | (HCl) | 248-250 |
| 5 | cis | H | Cyclohexyl | H | H | – | 131-134 |
| 6 | trans | H | Cyclohexyl | H | H | – | 146-148 |
| 7 | trans | H | Phenyl | H | H | – | 147-149 |
| 8 | cis | H | Phenyl | H | H | – | 135-137 |
| 9 | trans | H | $-CH(CH_3)_2$ | H | H | – | 125-127 |
| 10 | cis | H | $-CH(CH_3)_2$ | H | H | – | 115-117 |
| 11 | cis | H | $-C(CH_3)_2CH_2CH(CH_3)_2$ | H | H | – | 172-175 |
| 12 | cis | H | $-C(CH_3)_2CH_2C(CH_3)_3$ | H | H | – | 174-176 |
| 13 | trans | H | $-CH_2-CH_2-O-CH_3$ | H | H | – | 73-75 |
| 14 | trans | H | H | $-N(CH_3)_2$ | H | – | |
| 15 | trans | H | Cl | H | H | – | 130-132 |
| 16 | trans | H | F | H | H | – | 114-115 |
| 17 | trans | H | Cl | Cl | H | – | 175-178 |
| 18 | trans | H | $-C_2H_5$ | H | H | – | |
| 19 | trans | H | $-CH_3$ | H | $-CH_3$ | – | |
| 20 | trans | H | $-CH_3$ | $CH_3$ | H | – | |
| 21 | trans | H | $-C_3H_7$-n | H | H | – | |
| 22 | trans | H | $-C_4H_9$-n | H | H | – | |
| 23 | trans | H | $-C_4H_9$-i | H | H | – | |
| 24 | trans | H | $-C_4H_9$-sek | H | H | – | |
| 25 | trans | H | $-C_5H_{11}$-n | H | H | – | |

| Beisp. Nr. | R (trans/cis) $-C(CH_3)_3$ | $R^1$ | $R^2$ | $R^3$ | $R^4$ | Salz (Säure) | Fp °C |
|---|---|---|---|---|---|---|---|
| 26 | trans | H | $-C_6H_{13}$-n | H | H | - | |
| 27 | trans | H | Br | H | H | - | |
| 28 | trans | H | Cl | H | H | - | |
| 29 | trans | H | Cl | H | H | - | |
| 30 | trans | H | -CH=CH-CH=CH- | | | | 120-123 |
| 31 | (trans)$CH_3$ | H | $-C(CH_3)_3$ | H | H | - | |
| 32 | (trans)$CH_3$ | H | $-CH(CH_3)_2$ | H | H | - | |
| 33 | (trans)$CH_3$ | H | $-C(CH_3)_2CH_2CH_3$ | H | H | - | |
| 34 | (trans)$CH_3$ | H | Cyclohexyl | H | H | - | |
| 35 | (trans)$CH_3$ | H | Phenyl | H | H | - | |
| 36 | (trans)$CH_3$ | H | F | H | H | - | |
| 37 | (trans)$CH_3$ | H | Cl | H | H | - | |
| 38 | (trans)$CH_3$ | H | Br | H | H | - | |
| 39 | (trans)$CH_3$ | H | $CH_3$ | H | $CH_3$ | - | |
| 40 | (trans)$CH_3$ | H | Cl | H | $CH_3$ | - | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate) Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder

Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 3 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 4 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 6 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 7 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 2 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäureharnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde N-Tridecyl-2,6-dimethylmorpholin (A) - bekannt aus DE-A-11 64 152 - verwendet.

Anwendungsbeispiel 1

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebensperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 1, 2, 3, 4, 6 und 7 bei der Anwendung als 0,05 %ige (Gew.%) Spritzbrühe eine bessere fungizide Wirkung zeigen (97 %) als der bekannte Vergleichswirkstoff A (65 %).

Anwendungsbeispiel 2

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten,

tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 1, 2 und 6 bei der Anwendung als ,05 %ige Spritzbrühe eine bessere fungizide Wirkung zeigen (97 %) als der bekannte Vergleichswirkstoff A (20 %).

## Patentansprüche

1. o-Aminomethylphenole der allgemeinen Formel I

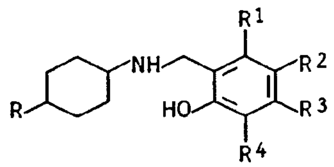

I

worin

$R^1$, $R^3$, $R^4$ Wasserstoff, Fluor, Chlor, Brom, $C_1$-$C_4$-Alkyl, $C_1$-$C_4$-Alkoxy oder Dimethylamino bedeuten und

$R^2$ Wasserstoff, Chlor, Brom, $C_1$-$C_{10}$-Alkyl, $C_3$-$C_8$-Alkenyl, Alkoxyalkyl, Cyclohexyl oder Phenyl bedeutet oder $R^1$ mit $R^2$, $R^2$ mit $R^3$ oder $R^3$ mit $R^4$ einen carbocyclischen, gesättigten oder ungesättigten Fünf- oder Sechsring bildet und

R $C_1$-$C_5$-Alkyl bedeutet, sowie deren Salze außer N-(4-tert.-Butyl-cyclohexyl)-3,5-dibrom-2-hydroxybenzylamin.

2. Verfahren zur Herstellung von o-Aminomethylphenolen gemäß Anspruch 1, dadurch gekennzeichnet, daß man

a) einen Salicylaldehyd der Formel II mit einem Amin der Formel III

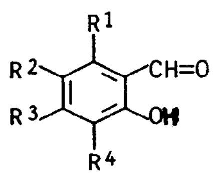

+

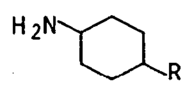

II III

in welcher R, $R^1$, $R^2$, $R^3$ und $R^4$ die oben genannten Bedeutungen haben, in Gegenwart von Ameisensäure, von Natriumcyanborhydrid, von Natriumborhydrid oder in Gegenwart von Wasserstoff und einem Hydrierkatalysator umsetzt, oder

b) ein Phenol der Formel IV mit Formaldehyd und einem Amin der Formel III

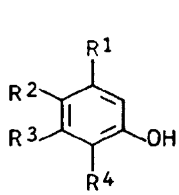

+ $CH_2=O$ +

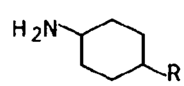

IV III

in welcher R, $R^1$, $R^2$, $R^3$ und $R^4$ die oben genannten Bedeutungen haben, in Gegenwart eines basischen Katalysators umsetzt und die erhaltenen Verbindungen gegebenenfalls in ihre Salze überführt.

3. Fungizide Mittel, enthaltend eine fungizid wirksame Menge eines o-Aminomethylphenols der allgemeinen Formel I

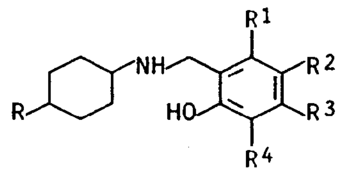

I

worin

$R^1$, $R^3$, $R^4$ Wasserstoff, Fluor, Chlor, Brom, $C_1$-$C_4$-Alkyl, $C_1$-$C_4$-Alkoxy oder Dimethylamino bedeuten, und

$R^2$ Wasserstoff, Chlor, Brom, $C_1$-$C_{10}$-Alkyl, $C_3$-$C_8$-Alkenyl, Alkoxyalkyl, Cyclohexyl oder Phenyl bedeutet oder $R^1$ mit $R^2$, $R^2$ mit $R^3$ oder $R^3$ mit $R^4$ einen carbocyclischen, gesättigten oder ungesättigten Fünf- oder Sechsring bildet und

R $C_1$-$C_5$-Alkyl bedeutet, oder dessen Salze außer N-(4-tert.-Butyl-cyclohexyl)-3,5-dibrom-2-hydroxy-benzylamin und einen festen oder flüssigen Trägerstoff.

4. Verfahren zur Herstellung von Fungiziden, dadurch gekennzeichnet, daß man eine Verbindung der Formel I gemäß Anspruch 1 mit festen oder flüssigen Trägerstoffen mischt.

5. Verfahren zur Bekämpfung von Pilzen, dadurch gekennzeichnet, daß man eine fungizid wirksame Menge eines o-Aminomethylphenols der allgemeinen Formel I

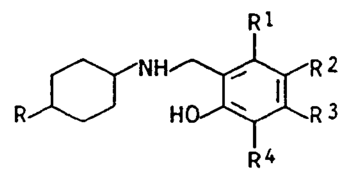

I

worin

$R^1$, $R^3$, $R^4$ Wasserstoff, Chlor, Brom, $C_1$-$C_{14}$-Alkyl, $C_1$-$C_4$-Alkoxy oder Dimethylamino bedeutet, und

$R^2$ Wasserstoff, Chlor, Brom, $C_1$-$C_{10}$-Alkyl, $C_3$-$C_8$-Alkenyl, Alkoxyalkyl, Cyclohexyl oder Phenyl bedeutet oder $R^1$ mit $R^2$, $R^2$ mit $R^3$ oder $R^3$ mit $R^4$ einen carbocyclischen, gesättigten oder ungesättigten Fünf- oder Sechsring bildet, und

R $C_1$-$C_5$-Alkyl bedeutet, oder dessen Salze auf die Pilze oder die von Pilzbefall bedrohten Flächen, Materialien, Pflanzen, Erdboden oder Saatgüter einwirken läßt.

6. Verbindung der Formel I gemäß Anspruch 1, in der $R^1$, $R^3$ und $R^4$ Wasserstoff, $R^2$ tert.-Pentyl und R tert.-Butyl bedeutet.

7. Verbindung der Formel I gemäß Anspruch 1, in der $R^1$, $R^3$ und $R^4$ Wasserstoff, $R^2$ und R tert.-Butyl bedeuten.

8. Verbindung der Formel I gemäß Anspruch 1, in der $R^1$, $R^3$ und $R^4$ Wasserstoff, $R^2$ Cyclohexyl und R tert.-Butyl bedeutet.

## Claims

1. An o-aminomethylphenol of the formula I

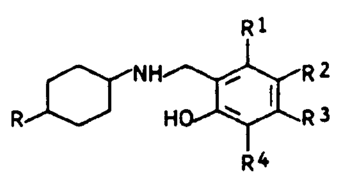

I

where

$R^1$, $R^3$ and $R^4$ are each hydrogen, fluorine, chlorine, bromine, $C_1$-$C_4$-alkyl, $C_1$-$C_4$-alkoxy or dimethylamino,

$R^2$ is hydrogen, chlorine, bromine, $C_1$-$C_{10}$-alkyl, $C_3$-$C_8$-alkenyl, alkoxyalkyl, cyclohexyl or phenyl or $R^1$

and $R^2$, $R^2$ and $R^3$ or $R^3$ and $R^4$ form a carbocyclic, saturated or unsaturated five-membered or six-membered ring and

R is $C_1$-$C_5$-alkyl, and salts thereof, with the exception of N-(4-tert-butylcyclohexyl )-3,5-dibromo-2-hydroxy-benzylamine.

2. A process for the preparation of an o-amino-methylphenol as claimed in claim 1, wherein

a) a salicylaldehyde of the formula II is reacted with an amine of the formula III

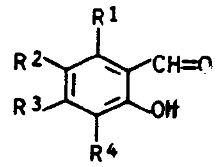
+
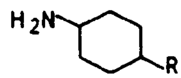

II III

where R, $R^1$, $R^2$, $R^3$ and $R^4$ have the abovementioned meanings, in the presence of formic acid, of sodium cyanoborohydride or of sodium borohydride or in the presence of hydrogen and a hydrogenation catalyst, or

b) phenol of the formula IV is reacted with formaldehyde and an amine of the formula III

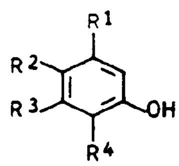
+ $CH_2=O$ +
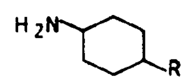

IV III

where R, $R^1$, $R^2$, $R^3$ and $R^4$ have the abovementioned meanings, in the presence of a basic catalyst, and the resulting compound is, if desired, converted into a salt

3. A fungicide containing a fungicidally effective amount of an o-aminomethylphenol of the formula I

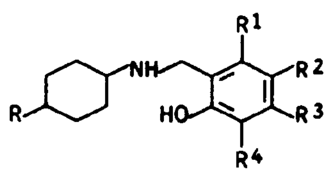

I

where

$R^1$, $R^3$ and $R^4$ are each hydrogen, fluorine, chlorine, bromine, $C_1$-$C_4$-alkyl, $C_1$-$C_4$-alkoxy or dimethylamino,

$R^2$ is hydrogen, chlorine, bromine, $C_1$-$C_{10}$-alkyl, $C_3$-$C_8$-alkenyl, alkoxyalkyl, cyclohexyl or phenyl or $R^1$ and $R^2$, $R^2$ and $R^3$ or $R^3$ and $R^4$ form a carbocyclic, saturated or unsaturated five-membered or six-membered ring and

R is $C_1$-$C_5$-alkyl, or salts thereof, with the exception of N-(4-tert-butylcyclohexyl)-3,5-dibromo-2-hydroxybenzylamine and a solid or liquid carrier.

4. A process for the preparation of a fungicide, wherein a compound of the formula I as claimed in claim 1 is mixed with a solid or liquid carriers.

5. A process for controlling fungi, wherein a fungicidally effective amount of an o-aminomethylphenol of the formula I

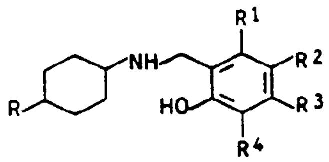

I

where

$R^1$, $R^3$ and $R^4$ are each hydrogen, chlorine, bromine, $C_1$-$C_4$-alkyl, $C_1$-$C_4$-alkoxy or dimethylamino, and

$R^2$ is hydrogen, chlorine, bromine, $C_1$-$C_{10}$-alkyl, $C_3$-$C_8$-alkenyl, alkoxyalkyl, cyclohexyl or phenyl or $R^1$ and $R^2$, $R^2$ and $R^3$ or $R^3$ and $R^4$ form a carbocyclic, saturated or unsaturated five-membered or six-membered ring, and

R is $C_1$-$C_5$-alkyl, or a salt thereof is allowed to act on the fungi or the areas, materials, plants, soil or seed threatened by fungal attack.

6. A compound of the formula I as claimed in claim 1, where $R^1$, $R^3$ and $R^4$ are each hydrogen, $R^2$ is tert-pentyl and R is tert-butyl.

7. A compound of the formula I as claimed in claim 1, where $R^1$, $R^3$ and $R^4$ are each hydrogen, and $R^2$ and R are each tert-butyl.

8. A compound of the formula I as claimed in claim 1, where $R^1$, $R^3$ and $R^4$ are each hydrogen, $R^2$ is cyclohexyl and R is tert-butyl.

## Revendications

1. o-aminométhylphénols de formule générale I

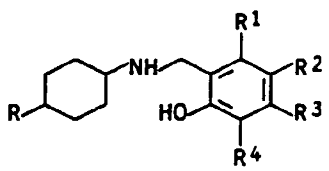

I

dans laquelle

$R^1$, $R^3$, $R^4$ représentent l'hydrogène, le fluor, le chlore, le brome, des groupes alkyle en C 1- C 4, alcoxy en C 1-C 4 ou diméthylamino,

$R^2$ représente l'hydrogène, le chlore, le brome un groupe alkyle en C 1-C 10, alcényle en C 3-C 8, alcoxyalkyle, cyclohexyle ou phényle ou bien $R^1$ et $R^2$, $R^2$ et $R^3$ ou $R^3$ et $R^4$ forment un noyau carbocyclique saturé ou insaturé à 5 ou 6 chaînons, et

R représente un groupe alkyle en C 1-C 5, et leurs sels, à l'exception de la N-(4-tert-butyl-cyclohexyl)-3,5-dibrome-2-hydroxy-benzylamine.

2. Procédé de préparation des o-aminométhylphénols de la revendication 1, caractérisé en ce que :

a) on fait réagir un salicylaldéhyde de forme II avec une amine de formule III

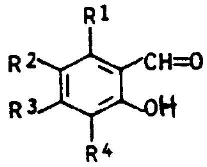

+

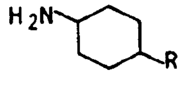

II III

dans lesquelles R, $R^1$, $R^2$, $R^3$ et $R^4$ ont les significations indiquées ci-dessus, en présence d'acide formique, du cyanoborohydrure de sodium, du borohydrure de sodium ou en présence d'hydrogène et d'un catalyseur d'hydrogénation, ou bien

b) on fait réagir un phénol de fomule IV avec le formaldéhyde et une amine de formule III

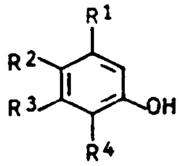

+ $CH_2=O$ +

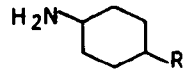

IV III

dans lesquelles R, $R^1$, $R^2$, $R^3$ et $R^4$ ont les significations indiquées ci-dessus, en présence d'un catalyseur basique, et le cas échéant on convertit les composés obtenus en leurs sels.

3. Produits fongicides contenant une quantité fongicide efficace d'un o-aminométhylphénol de formule générale I

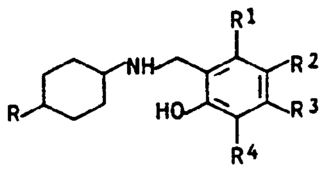

I

dans laquelle

$R^1$, $R^3$, $R^4$ représentent l'hydrogène, le fluor, le chlore, le brome, des groupes alkyle en C 1-C 4, alcoxy en C 1-C 4 ou diméthylamino, et

$R^2$ représente l'hydrogène, le chlore, le brome, un groupe alkyle en C 1-C 10, alcényle en C 3-C 8, alcoxyalkyle, cyclohexyle ou phényle ou bien $R^1$ et $R^2$, $R^2$ et $R^3$ ou $R^3$ et $R^4$ forment ensemble un noyau carbocyclique saturé ou insaturé à 5 ou 6 chaînons, et

R représente un groupe alkyle en C 1- C 5, ou de l'un de ses sels, à l'exception de la N-(4-tert-butylcyclohexyl)-3,5-dibrome-2-hydroxy-benzylamine, et un véhicule solide ou liquide.

4. Procédé de préparation de fongicides, caractérisé en ce que l'on mélange un composé de formule I de la revendication 1 avec des véhicules solides ou liquides.

5. Procédé pour combattre les mycètes, caractérisé en ce que l'on fait agir sur les mycètes ou sur les surfaces, matériaux, végétaux, sols ou semences menacés d'une attaque par les mycètes, une quantité fongicide efficace d'un o-aminométhylphénol de formule générale I

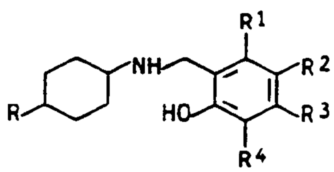

I

dans laquelle

$R^1$, $R^3$, $R^4$ représentent l'hydrogène, le chlore, le brome, des groupes alkyle en C 1-C 4, alcoxy en C 1-C 4 ou diméthylamino, et

$R^2$ représente l'hydrogène, le chlore, le brome, un groupe alkyle en C 1-C 10, alcényle en C 3-C 8, alcoxyalkyle, cyclohexyle ou phényle ou bien $R^1$ et $R^2$, $R^2$ et $R^3$ ou $R^3$ et $R^4$ forment ensemble un noyau carbocyclique saturé ou insaturé à 5 ou 6 chaînons, et

R représente un groupe alkyle en C 1-C 5, ou de l'un de ses sels.

6. Composé de formule I de la revendication 1, dans laquelle $R^1$, $R^3$ et $R^4$ représentent l'hydrogène, $R^2$ un groupe tert-pentyle et R un groupe tert-butyle.

7. Composé de formule I de la revendication 1, dans laquelle $R^1$, $R^3$ et $R^4$ représentent l'hydrogène, $R^2$ et R des groupes tert-butyle.

8. Composé de formule I de la revendication 1, dans laquelle $R^1$, $R^3$ et $R^4$ représentent l'hydrogène, $R^2$ un groupe cyclohexyle et R un groupe tert-butyle.